(19) Europäisches Patentamt
European Patent Office
Office européen des brevets

(11) Veröffentlichungsnummer: **0 181 561**
**A1**

(12) # EUROPÄISCHE PATENTANMELDUNG

(21) Anmeldenummer: **85113776.0**

(22) Anmeldetag: **29.10.85**

(51) Int. Cl.[4]: **C 07 J 51/00**

(30) Priorität: **08.11.84 DE 3440794**

(43) Veröffentlichungstag der Anmeldung:
**21.05.86 Patentblatt 86/21**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Engels, Joachim, Prof. Dr.**
**Feldbergstrasse 1**
**D-6242 Kronberg/Taunus(DE)**

(54) **Verfahren zur Herstellung von Corticosteroid-21-phosphorsäuren und deren Salzen und die Corticosteroid-21-phosphorsäuretriester.**

(57) Steroid-21-phosphorsäuren und ihre pharmazeutisch brauchbaren Salze der Formel III

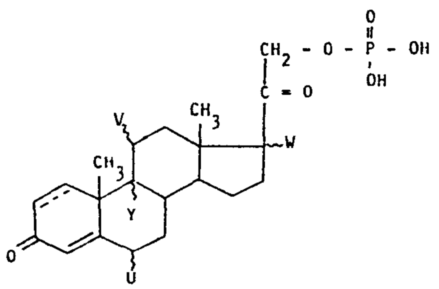

(mit U= H, $CH_3$; V= H, OH, O, Hal; W= H, OH und Y= H oder F) erhält man in sehr reiner Form durch Umsetzung von I

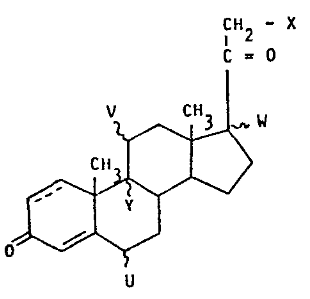

I,

Mit X= OH oder Hal mit einem organischen Phosphorsäureester der Formel IVa oder IVb

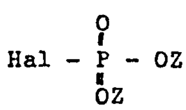

IVa

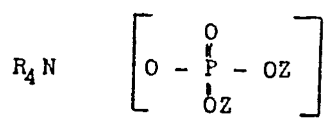

IVb

mit Z= gegebenenfalls substituiertem Alkyl und R= Alkyl). Die dabei resultierende Verbindung II

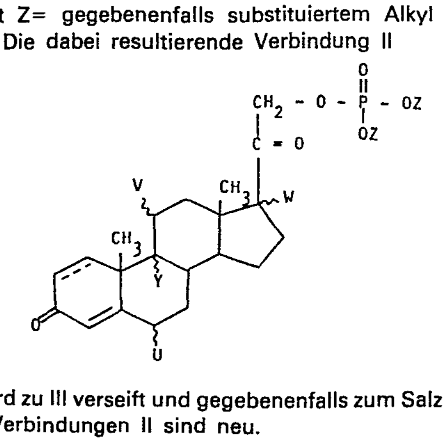

wird zu III verseift und gegebenenfalls zum Salz neutralisiert. Verbindungen II sind neu.

EP 0 181 561 A1

Croydon Printing Company Ltd.

0181531

HOECHST AKTIENGESELLSCHAFT HOE 84/F 261 Dr.v.F./St

# Verfahren zur Herstellung von Corticosteroid-21-phosphorsäuren und deren Salzen und die Corticosteroid-21-phosphorsäuretriester

Die vorliegende Erfindung betrifft ein Verfahren zur Herstellung von Corticosteroid-21-phosphorsäuren sowie von deren pharmazeutisch wirksamen Salzen, insbesondere von Methylprednisolon-di-natriumphosphat, sowie die Corticosteroid-21-phosphorsäuretriester.

Steroid-21-phosphate und deren pharmazeutisch wirksame Salze sind aus der japanischen Auslegeschrift Sho 41-12351, der US-Patentschrift 2 932 657, der deutschen Offenlegungsschrift 2 225 658 und aus der britischen Patentschrift 1 010 031 bekannt. Ein empfindlicher Nachteil der bekannten Herstellungsverfahren besteht darin, daß z.B. ein beachtlicher Anteil an Phosphorsäurediester entsteht und daß es aufgrund der Reaktionsbedingungen (Phosphatüberschuß) sehr schwierig ist, ein von Fremdsalzen salzfreies Steroidphosphat herzustellen.

Die Erfindung hat sich deshalb die Aufgabe gestellt, Corticosteroid-21-phosphorsäuren sowie deren pharmazeutisch wirksame Salze auf einfache Art in hochreiner Form darzustellen.
Überraschenderweise ist es gelungen, diese Verbindungen der Formel III

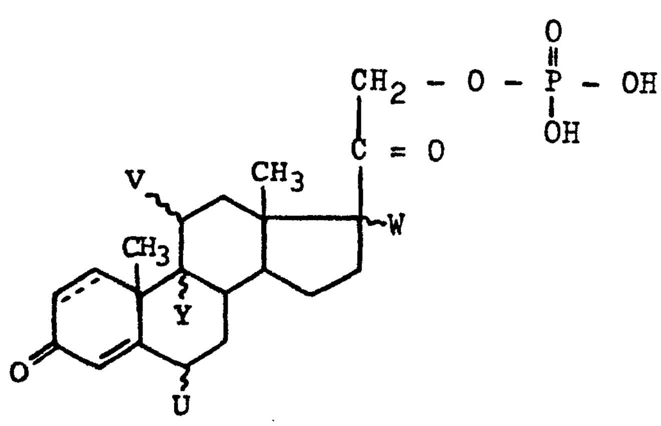

III,

in der

U H, $CH_3$,

V H, OH, O, Halogen

W H, OH und

Y H oder F bedeuten,

oder deren pharmazeutisch wirksame Salze, dadurch herzustellen, daß man ein Corticosteroid der Formel I

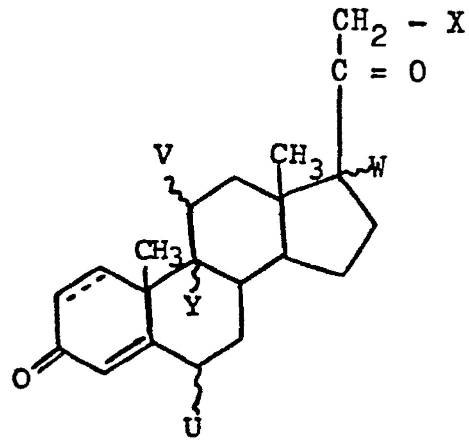

I,

in der U, V, W und Y die angegebene Bedeutung haben und X für OH oder Halogen steht, mit einem organischen Phosphorsäureester der Formel IVa bzw. IVb umsetzt

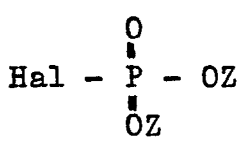

IVa

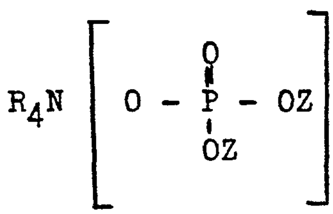

IVb

in der

Z $C_1$-$C_8$-, vorzugsweise $C_1$-$C_4$-Alkyl ist, das in α-Stellung mit Phenyl substituiert sein kann, das seinerseits mit Cl, Br, CN oder $NO_2$ substituiert sein kann und wobei Alkylreste mit mindestens 2 C-Atomen in ß-Stellung einfach mit CN, Nitrophenyl oder $SO_2$-$C_{1-4}$-Alkyl oder bis zu dreifach mit Cl und/oder Br substituiert sein können, und

Hal Halogen, vorzugsweise Cl oder Br ist, und

R $C_1$-$C_8$-Alkyl bedeutet, jedoch ein Rest R auch Benzyl und/oder Wasserstoff sein kann,

und so eine neue Verbindung der Formel II herstellt,

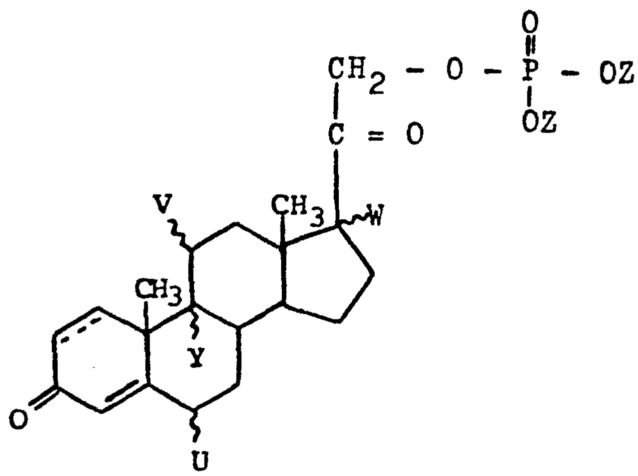

II,

in der U, V, W, Y und Z die genannten Bedeutungen haben, und diese Verbindung II zur Verbindung III verseift, und daß man gegebenenfalls zum Salz neutralisiert.

Sofern Z substituierte Phenylreste enthält, befinden sich die Substituenten bevorzugt in o- oder p-Stellung.

Zur Herstellung der Verbindungen II haben sich insbesondere Verfahren bewährt, bei denen eine Verbindung der Formel I, in der X Hydroxy bedeutet, mit einer Verbindung IV umgesetzt werden, in der Hal Chlor bedeutet. Ferner ist vielfach ein Verfahren bevorzugt, bei dem Z tert.-Butyl bedeutet. Ebenfalls bevorzugt ist ein Verfahren, bei welchem eine Verbindung der Formel I mit X = Br oder J mit einem ($C_1$-$C_8$)-Alkyl- bzw. Aralkylammoniumsalz einer ($C_1$-$C_4$)-Dialkylphosphorsäure umgesetzt wird.

Die Verseifung der Verbindungen II kann mit Säuren oder Basen erfolgen. Vielfach wird sie mit besonderem Vorteil mit Säuren durchgeführt, die einen $p_K$-Wert von kleiner als 3 haben, bevorzugt Salzsäure oder Trifluoressigsäure.

Halogen bedeutet, sofern nicht ausdrücklich anders angegeben, Fluor, Chlor, Brom oder Jod.

Das erfindungsgemäße Verfahren umfaßt beispielsweise folgende Ausführungsformen: Umsetzen eines Hydroxy-cortico-steroids, insbesondere 6α-Methylprednisolon mit einem organischen Phosphorsäurediesterchlorid, z.B. Di-tert.butylphosphorsäurechlorid in Gegenwart einer Base, z.B. Pyridin. Alternativ kann z.B. vom 21-Jod-prednisolon ausgegangen werden, das mit einem Alkylammoniumsalz eines organischen Phosphorsäurediesters in einem inerten Lösungsmittel wie Methylenchlorid, Acetonitril oder einem Ether wie Dimethoxyethan umgesetzt wird. Der resultierende Corticosteroidphosphorsäuretriester wird nach Extraktion in ein organisches Lösungsmittel, z.B. Methylenchlorid, mit Wasser gewaschen und nach Trocknen der organischen Phase auskristallisiert und so von wasserlöslichen und anderen organischen Verunreinigungen getrennt.

In der nächsten Stufe wird der neue Steroidphosphorsäuretriester II mit einer Säure, z.B. HCl oder Trifluoressigsäure, in den Corticosteroidphosphorsäuremonoester übergeführt, vorzugsweise in einem inerten Lösungsmittel, wie Chloroform, halogenierte Kohlenwasserstoffe oder Methylenchlorid bei Zimmertemperatur. Bei Temperaturen oberhalb von 40°C sinken die Ausbeuten, da das Steroidgerüst zunehmend unbeständig wird. Hierbei fällt das Corticosteroidphosphat bereits in sehr guter Reinheit an. Die Verseifung des Steroidphosphorsäuretriesters zum Stereoidphosphorsäuremonoester kann auch alkalisch vorgenommen werden, wenn der Steroid-Rest nicht alkaliempfindlich ist. Nach Entfernen des Lösungsmittels und der überschüssigen Säure wird eine Verteilung zwischen a) einer organischen Phase, wie zunächst Methylenchlorid, dann z.B. n-Butanol oder n-Hexanol, und b) Wasser vorgenommen. In der organischen Phase wird dann durch Titration mit einer Base, vorzugsweise Natronlauge, der pH der Lösung auf schwach basisch eingestellt und so z.B. das Dinatriumsalz gewonnen. Dieses geht dabei in die wässerige Phase über und letzte organische Verunreinigungen bleiben in der organischen Phase.

Das erhaltene Steroid-21-phosphat kann entweder durch Verdampfen oder durch Gefriertrocknung der wässerigen Lösung, falls erwünscht, nach weiterer Reinigung mit Aktivkohle, in fester Form erhalten werden.

Als Ausgangs-Corticosteroide sind von besonderem Interesse das Cortisol, Cortison, Prednisolon, 6α-F-Prednisolon, Dexamethason, Betamethason, Desoximethason, 6α-Methylprednisolon und ähnliche.

Gegenstand der Erfindung sind auch die in der ersten Stufe erhaltenen neuen Verbindungen der Formel II.

Beispiele

1A) 7,5 g 6α-Methylprednisolon, in 35 ml wasserfreiem Pyridin und 8 ml Triethylamin gelöst, wurden bei -40°C mit 10 g Ditertiärbutylphosphorsäurechlorid, in 30 ml Methylenchlorid gelöst, innert 20 Minuten versetzt. Die Reaktion wurde bei -20°C für 40-60 Stunden geführt. Nach Abziehen des Lösungsmittels wurde der Rückstand zwischen 120 ml Methylenchlorid und 40 ml Wasser verteilt. Nach Waschen der organischen Phase mit Wasser und Trocknen mit Natriumsulfat kristallisierte das resultierende Öl mit Hilfe von Essigester. Man erhielt 7,8 g des neuen Ditertiärbutylesters vom Schmp. 153°C (Zers.)

$[\alpha_D]^{23}$ = +70.8° C,H,P ber. C 63,6 H 8,3 P 5,4 %
gef. C 63,2 H 8,3 P 5,2 %

$^{31}$P-NMR ($d_6$DMSO) rel. 85 % $H_3PO_4$ $\delta$ = 8,9 ppm

IR (KBr): $\nu$ = 2980, 2930, 1740, 1650, 1610, 1370, 1260, 1000 $cm^{-1}$

$^{1}$H-NMR: ($CDCl_3$/TMS) $\delta$ = 1,5 ppm $[C-(CH_3)_3]$

1B) 21-Jod-6α-methylprednisolon 9,7 g und 15 g Tetra-n-butylammonium-bis-tertiärbutylphosphat wurden in 150 ml säurefreiem Methylenchlorid gelöst und bei Raumtemperatur über 20 Stunden gerührt. Die Reaktionsmischung wurde anschließend mit Wasser, einer 2%igen Thiosulfatlösung, gefolgt von Wasser, gewaschen und getrocknet. Nach Entfernen des Methylenchlorids kristallisierte der 6α-Methylprednisolon-21-bis-tertiärbutylphosphorsäureester aus Essigester; 11 g vom Schmp. 150-152°C (Zers.)
analyt. Daten s. Beispiel 1 A.

1C) 5,3 g 21-Jod-6α-methylprednisolon und 9 g Benzyltriethylammonium-ditertiärbutylphosphat wurden in 135 ml Dimethoxyethan gelöst und bei Raumtemperatur über 40 Stunden unter Lichtausschluß gerührt. Nach Entfernen des Lösungsmittels wurde zwischen Methylenchlorid und Wasser verteilt, mit Thiosulfat gewaschen und die organische Phase mit Natriumsulfat getrocknet. Mit Hilfe von Essigester kristallisierte der 6-α-Methylprednisolon-21-bis-tertiärbutylphosphorsäureester aus, 5,4 g dessen analytische Daten mit denen von Beispiel 1A und 1B identisch sind.

2) 3,7 g 6α-Methylprednisolon, in 35 ml wasserfreiem Pyridin gelöst, wurden bei 0°C mit 1,7 g Phosphorsäuredimethylesterchlorid, gelöst in 25 ml Methylenchlorid, innerhalb 30 Minuten versetzt. Nach 18 Stunden bei Zimmertemperatur war die Reaktion beendet; das Pyridin wurde unter vermindertem Druck entfernt. Der Rückstand wurde zwischen Methylenchlorid und Wasser (pH 3) verteilt, mit Wasser neutral gewaschen und die organische Phase mit $Na_2SO_4$ getrocknet. Nach Entfernen des Lösungsmittels wurde der verbleibende neue 6α-Methylprednisolon-21-bismethylphosphorsäureester (3,1 g) aus Dioxan/Dimethylformamid (8:1) umkristallisiert. Schmp. 245-46°C (Zers.)

$^{31}$P-NMR ($d_6$DMSO) rel. 85 % $H_3PO_4$ δ= 2,3 ppm
$^{1}$H-NMR ($d_6$DMSO) δ= 3,62 (Dublett) ppm ($P-OCH_3$)

3) 3,7 g 6α-Methylprednisolon, in 42 ml wasserfreiem Pyridin gelöst, wurden mit 6,0 g Bis-2,2,2-trichlorethyl-phosphorsäurechlorid bei Zimmertemperatur versetzt. Nach 20 Stunden wurde das Reaktionsgemisch aufgearbeitet, Pyridin unter vermindertem Druck abdestilliert, der Rückstand zwischen Methylenchlorid und Wasser (pH 2) verteilt, dann mit Wasser neutral gewaschen und die organische Phase mit $Na_2SO_4$ getrocknet. Es resultierten 1,6 g des neuen 6α-Methylprednisolon-21-bis-2,2,2-trichlorethylphosphorsäureesters in Form farbloser Kristalle vom Schmp. 216-217°C.
$^{31}P$-NMR ($d_6$DMSO) rel. 85 % $H_3PO_4$ δ= 9,4 ppm

4) 3,7 g 6α-Methylprednisolon in 40 ml wasserfreiem Pyridin wurden bei 0°C mit 6,3 g Bis-4-nitrophenylethyl-phosphorsäurechlorid versetzt, das Gemisch 2 Stunden bei 0°C gehalten und weitere 3 Stunden bei Zimmertemperatur stehengelassen. Nach Entfernen des Pyridins wurde der Rückstand zwischen Methylenchlorid und Wasser (pH 3) verteilt, mit Wasser nachgewaschen und die organische Phase mit $Na_2SO_4$ getrocknet. Das Rohprodukt wurde chromatographisch an 300 g Kieselgel mit 3 % Methanol in Methylenchlorid als Eluens gereinigt. Der neue 6α-Methylprednisolon-21-bis-4-nitrophenylethylphosphorsäureester (5,7 g) kristallisierte aus Toluol/Diethylether (4:1). Schmp. 174-176°C.
UV (Methanol): $\varepsilon_{260}$ = 16000 $l \times Mol^{-1} \times cm^{-1}$

5) 10,8 g des gemäß 1A, 1B oder 1C hergestellten Esters wurden in 190 ml Chloroform gelöst und nach Zugabe von 10,8 g (7,35 ml) Trifluoressigsäure für 20 Stunden bei Raumtemperatur gerührt. Lösungsmittel sowie Trifluoressigsäure wurden unter vermindertem Druck entfernt, eventuell mit Hilfe von Toluol. Der Rückstand wurde zwischen destilliertem Wasser und Chloroform verteilt und anschließend zwischen Wasser und n-Hexanol. Das Steroidphosphat befand

sich dann in n-Hexanol. Die n-Hexanolphase wurde mit frischem destilliertem Wasser unterschichtet und dann die Mischung vorsichtig mit 1N Natronlauge auf pH 8,6 titriert. Die wässerige Phase wurde abgetrennt, nochmals mit Diethylether gewaschen und das Dinatriumsalz der 6a-Methylprednisolon-21-phosphorsäure durch Gefriertrocknung als farbloses Pulver erhalten.

$[\alpha]_D^{24} = 84°$ ($H_2O$) $^{31}P$-NMR ($D_2O$) $\delta = 3,5$ ppm

UV: $\varepsilon_{243} = 12600 \; 1 x Mol^{-1} x cm^{-1}$

6) 7,5 g 6α-Methylprednisolon-21-bis-4-nitrophenylethylphosphorsäureester gemäß Beispiel 4 wurden in 500 ml Diazabicycloundecen in Pyridin (0,5 molar) bei Raumtemperatur gelöst und das Gemisch 40 Stunden gerührt. Nach Entfernen des Lösungsmittels wurde der Rückstand zwischen Methylenchlorid und Wasser verteilt; die organischen Extrakte wurden verworfen. Anschließend wurde die Lösung mit Salzsäure auf pH 1 gebracht und das Produkt in n-Butanol oder n-Hexanol extrahiert. Die Butanol- bzw. Hexanolphase wurde mit destilliertem Wasser unterschichtet und dann die Mischung mit 1N NaOH auf pH 8,5 titriert. Die wäßrige Phase wurde abgetrennt, nochmals mit Diethylether gewaschen und das Dinatriumsalz des 6α-Methylprednisolon-21-phosphates durch Gefriertrocknung als farbloses Pulver erhalten. Es war mit dem von Beispiel 5 identisch.

PATENTANSPRÜCHE:

1. Verfahren zum Herstellen von Corticosteroid-21-phosphorsäuren der allgemeinen Formel III

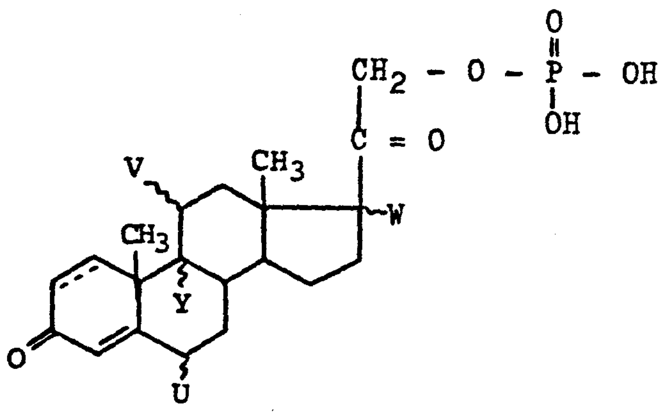

II

und von deren pharmazeutisch wirksamen Salzen,
in welcher Formel III
U H, $CH_3$,
V H, OH, O, Halogen
W H, OH und
Y H oder F bedeuten,
dadurch gekennzeichnet, daß man ein Corticosteroid der Formel I

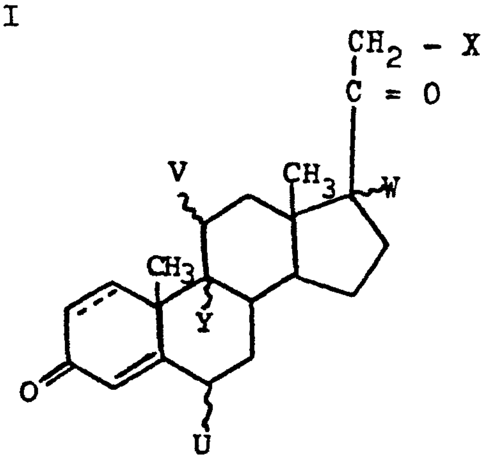

I,

in der U, V, W und Y die angegebene Bedeutung haben und X für OH oder Halogen steht,
mit einem organischen Phosphorsäureester der Formel IVa oder IVb umsetzt

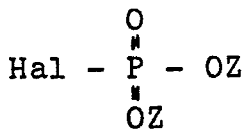

IVa

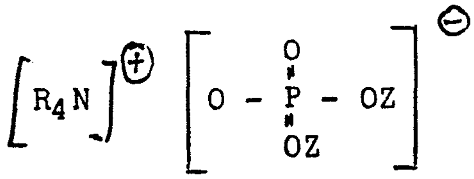

IVb

in der

Z $C_{1-8}$-Alkyl ist, das unsubstituiert oder in α-Stellung mit Phenyl substituiert ist, das seinerseits unsubstituiert oder mit Cl, Br, CN oder $NO_2$ substituiert ist, und wobei Alkylreste mit mindestens 2 C-Atomen in ß-Stellung unsubstituiert oder einfach mit CN, Nitrophenyl oder $SO_2$-$C_{1-4}$-Alkyl oder bis zu dreifach mit Cl und/oder Br substituiert sind, und

Hal Halogen und

R $C_{1-8}$-Alkyl bedeutet, jedoch ein Rest R auch Benzyl und/oder Wasserstoff sein kann,

und so eine Verbindung der Formel II herstellt,

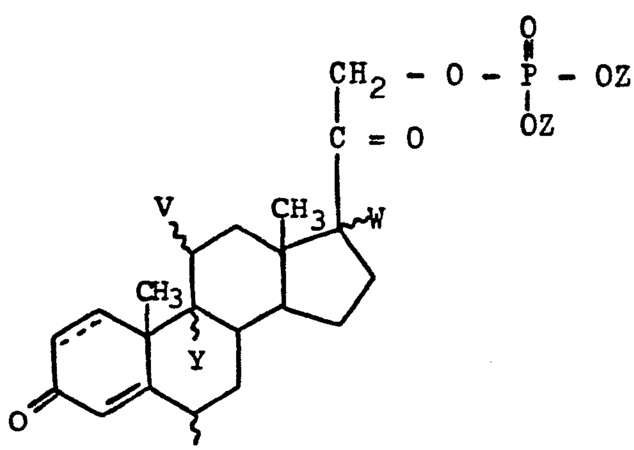

II

in der U, V, W, Y und Z die genannten Bedeutungen haben, und diese Verbindung II zur Verbindung III verseift, und daß man gegebenenfalls zum Salz neutralisiert.

2. Verfahren nach Anspruch 1, dadurch gekennzeichnet, daß X Hydroxy und Hal Chlor bedeuten.

3. Verfahren nach Anspruch 1, dadurch gekennzeichnet, daß X Brom oder Jod bedeutet und daß die Verbindung der Formel I mit einem ($C_1$-$C_8$)-Alkyl- bzw. Aralkylammoniumsalz einer ($C_1$-$C_4$)-Dialkylphosphorsäure umgesetzt wird.

4. Verfahren nach einem oder mehreren der Ansprüche 1 bis 3, dadurch gekennzeichnet, daß die Verseifung mit einer Säure mit einem $p_K$-Wert von kleiner als 3, vorzugsweise mit Salzsäure oder Trifluoressigsäure, durchgeführt wird.

5. Verbindungen der Formel II wie im Anspruch 1 definiert.

6. Ausführungsform nach einem oder mehreren der Ansprüche 1 bis 5, dadurch gekennzeichnet, daß, sofern Z substituierte Phenylreste enthält, sich diese in o- oder p-Stellung befinden.

7. Ausführungsform nach einem oder mehreren der Ansprüche 1 bis 5, dadurch gekennzeichnet, daß Z $C_1$-$C_4$-Alkyl, insbesondere tert.-Butyl ist.

Patentansprüche Österreich:

1. Verfahren zum Herstellen von Corticosteroid-21-phosphorsäuren der allgemeinen Formel III

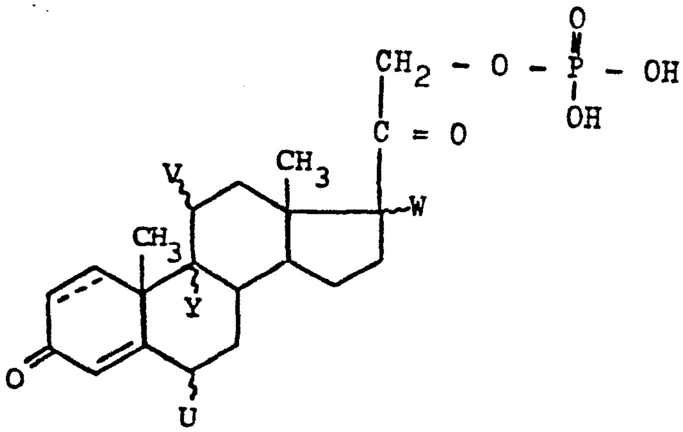

III,

und von deren pharmazeutisch wirksamen Salzen, in welcher Formel III

U H, $CH_3$,

V H, OH, O, Halogen

W H, OH und

Y H oder F bedeuten,

dadurch gekennzeichnet, daß man ein Corticosteroid der Formel I

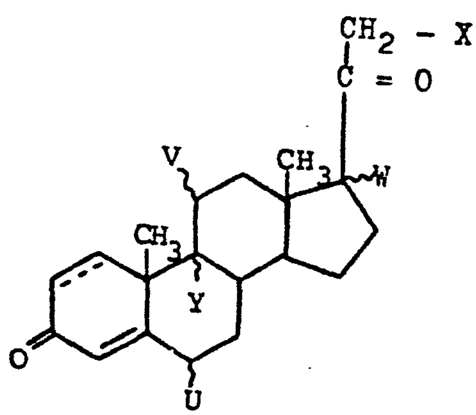

I,

in der U, V, W und Y die angegebene Bedeutung haben und X für OH oder Halogen steht, mit einem organischen Phosphorsäureester der Formel IVa oder IVb umsetzt

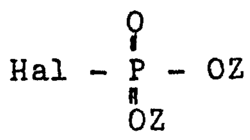

IVa

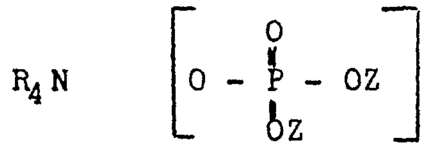

IVb

in der

Z $C_{1-8}$-Alkyl ist, das unsubstituiert oder in α-Stellung mit Phenyl substituiert ist, das seinerseits unsubstituiert oder mit Cl, Br, CN oder $NO_2$ substituiert ist, und wobei Alkylreste mit mindestens 2 C-Atomen in ß-Stellung unsubstituiert oder einfach mit CN, Nitrophenyl oder $SO_2$-$C_{1-4}$-Alkyl oder bis zu dreifach mit Cl und/oder Br substituiert sind, und

Hal Halogen und

R $C_{1-8}$-Alkyl bedeutet, jedoch ein Rest R auch Benzyl und/oder Wasserstoff sein kann,

und so eine Verbindung der Formel II herstellt,

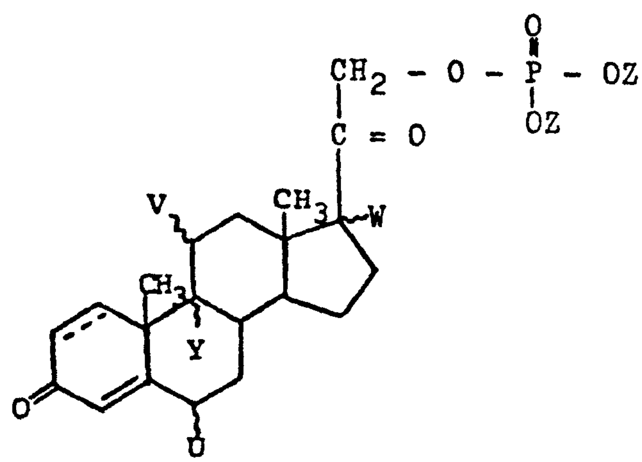

II,

in der U, V, W, Y und Z die genannten Bedeutungen haben, und diese Verbindung II zur Verbindung III verseift, und daß man gegebenenfalls zum Salz neutralisiert.

2. Verfahren nach Anspruch 1, dadurch gekennzeichnet, daß X Hydroxy und Hal Chlor bedeuten.

3. Verfahren nach Anspruch 1, dadurch gekennzeichnet, daß X Brom oder Jod bedeutet und daß die Verbindung der Formel I mit einem $(C_1-C_8)$-Alkyl- bzw. Aralkyl-ammoniumsalz einer $(C_1-C_4)$-Dialkylphosphorsäure umgesetzt wird.

4. Verfahren nach einem oder mehreren der Ansprüche 1 bis 3, dadurch gekennzeichnet, daß die Verseifung mit einer Säure mit einem $p_K$-Wert von kleiner als 3, vorzugsweise mit Salzsäure oder Trifluoressigsäure, durchgeführt wird.

5. Verfahren zum Herstellen von Verbindungen der Formel II

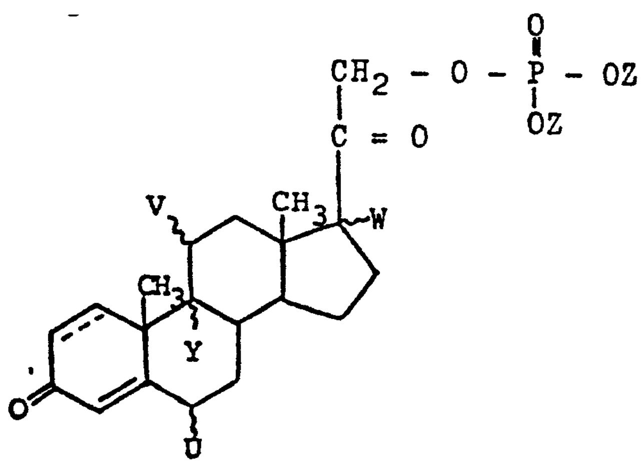

II,

in der

U H, $CH_3$

V H, OH, O, Halogen

W H, OH

Y H oder F und

Z $C_{1-8}$-Alkyl ist, das unsubstituiert oder in α-Stellung mit Phenyl substituiert ist, das seinerseits unsubstituiert oder mit Cl, Br, CN oder $NO_2$ substituiert ist, und wobei Alkylreste mit mindestens 2 C-Atomen in ß-Stellung unsubstituiert oder einfach mit CN, Nitrophenyl oder $SO_2$-$C_{1-4}$-Alkyl oder bis zu dreifach mit Cl und/

oder Br substituiert sind,
bedeuten,
dadurch gekennzeichnet, daß man ein Corticoid der Formel I

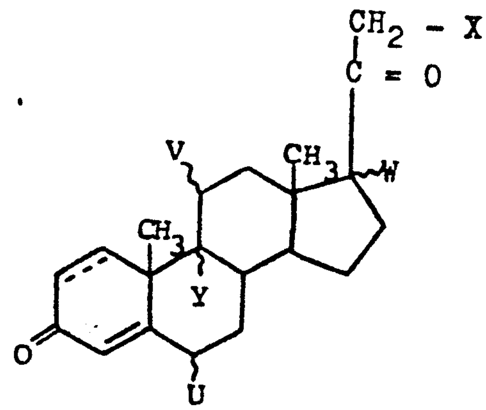

I,

in der U, V, W undY die angegebene Bedeutung haben und X für OH oder Halogen steht,
mit einem organischen Phosphorsäureester der Formel IVa oder IVb umsetzt

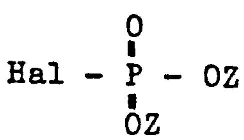

IVa

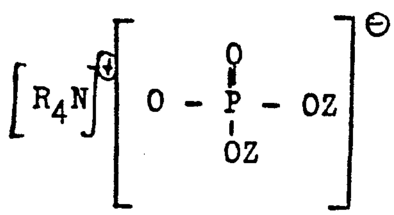

IVb

in der
Z die angegebene Bedeutung hat,
Hal Halogen bedeutet, und
R $C_{1-8}$-Alkyl bedeutet, jedoch ein Rest R auch Benzyl und/oder Wasserstoff sein kann.

6. Ausführungsform nach einem oder mehreren der Ansprüche 1 bis 5, dadurch gekennzeichnet, daß, sofern Z substituierte Phenylreste enthält, sich diese in o- oder p-Stellung befinden.

7. Ausführungsform nach einem oder mehreren der Ansprüche 1 bis 5, dadurch gekennzeichnet, daß Z $C_1$-$C_4$-Alkyl, insbesondere tert.-Butyl ist.

0181561

Europäisches Patentamt

**EUROPÄISCHER RECHERCHENBERICHT**

Nummer der Anmeldung

EP 85 11 3776

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | DE-A-1 493 330 (UPJOHN)<br>* Insgesamt *<br>--- | 1 | C 07 J 51/00 |
| A | US-A-3 248 408 (JAMES S. GRIER)<br>----- | | |
| | | | RECHERCHIERTE SACHGEBIETE (Int. Cl.4) |
| | | | C 07 J 51/00 |

Der vorliegende Recherchenbericht wurde für alle Patentansprüche erstellt.

| Recherchenort | Abschlußdatum der Recherche | Prüfer |
|---|---|---|
| DEN HAAG | 20-02-1986 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82